# EUROPEAN PATENT SPECIFICATION

(11) **EP 3 886 907 B1**
(45) Date of publication and mention of the grant of the patent: **02.10.2024**
(21) Application number: 19804610.4
(22) Date of filing: 25.11.2019
(51) Int. Cl.: A61K 9/08, A61K 47/06, A61K 31/232, A61P 27/04

(54) **SEMIFLUORINATED ALKANE COMPOSITIONS COMPRISING OMEGA-3 FATTY ACID ETHYL ESTERS**
SEMIFLUORIERTE ALKANZUSAMMENSETZUNGEN MIT OMEGA-3-FETTSÄUREETHYLESTERN
COMPOSITIONS D'ALCANE SEMIFLUORÉ COMPRENANT DES ESTERS ÉTHYLIQUES D'ACIDES GRAS OMÉGA-3

(30) Priority: 27.11.2018 EP 18208650; 09.08.2019 EP 19191084
(43) Date of publication of application: 06.10.2021
(73) Proprietor: Novaliq GmbH, 69120 Heidelberg (DE)
(72) Inventor: MAUDEN, Jörg, Martin, 69121 Heidelberg (DE); STICHER, Anja, 67549 Worms (DE); FRANZINI, Emanuele, 74177 Bad Friedrichshall (DE); LÖSCHER, Frank, 69198 Schriesheim (DE); COORS, Michael, 64319 Pfungstadt (DE)
(74) Representative: Synergy IP Group AG
(86) International application number: PCT/EP2019/082338
(87) International publication number: WO 2020/109192

(56) References cited:
- WO-A1-2014/041071
- WO-A1-2017/066429

## Description

### FIELD

The present invention is in the field of compositions comprising omega-3 fatty acid ethyl esters, specifically compositions that are based on semifluorinated alkanes. In particular, the compositions of the present invention may be topical ophthalmic compositions useful in the treatment of ocular diseases or conditions.

### BACKGROUND

Omega-3 fatty acid compounds are useful in the field of pharmaceutics and therapeutic applications. The formulation of these compounds and long-term storage of these compounds due to their susceptibility towards chemical degradation by oxidation and other pathways is generally of concern. The loss and aging of the compositions in particular over a long-term period storage are often addressed by the use of addition of antioxidants and other means to reduce the chemical instability of these compounds. However, the long-term stability of a pharmaceutical composition is not only affected by chemical stability of the active agent, but also the physical stability of the composition as such.

In WO 2014/041071, compositions comprising active compounds prone to oxidation, including polyunsaturated fatty acids such as omega-3 and omega-6 fatty acids and derivatives thereof, dissolved, dispersed or suspended in a liquid vehicle comprising a semifluorinated alkane are described. Solution compositions of omega-3 fatty acid ethyl esters such as eicosapentaenoic acid ethyl ester and docosahexaenoic acid ethyl ester and mixtures thereof in a semifluorinated alkane are described.

It has been observed that liquid compositions comprising an omega-3 fatty acid ester and a semifluorinated alkane under extended storage conditions, may lead to the formation of deposits of the omega-3 fatty acid ester along the walls of the containers in which the compositions are stored, in particular at the head-space-liquid interface in the form of tidemarks, may occur.

As a physical removal of the omega-3 fatty acid ester from the composition to container walls in which it is stored may affect, for example, the accurate dosing of the active agent, there is a need to minimize and reduce such potential losses.

It is therefore an objective of the present invention, to provide a composition which provides an improved formulation for long-term storage over the prior art It is a further objective of the invention, to provide compositions for ophthalmic use and for use in the treatment of ophthalmic conditions such as keratoconjunctivitis sicca, meibomian gland dysfunction or a symptom associated therewith. Further objectives of the invention will become clear on the basis of the description, example and claims below.

### BRIEF DESCRIPTION OF THE DRAWINGS

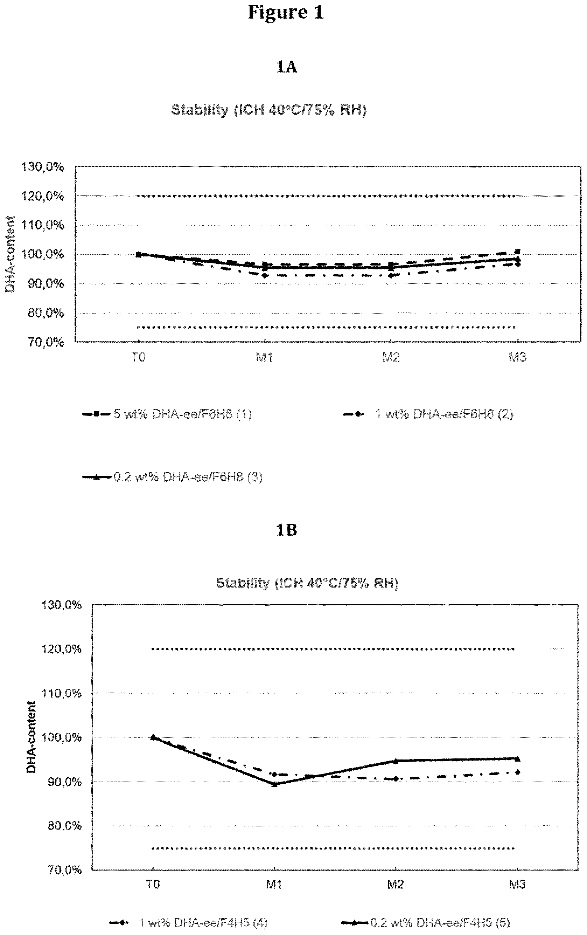

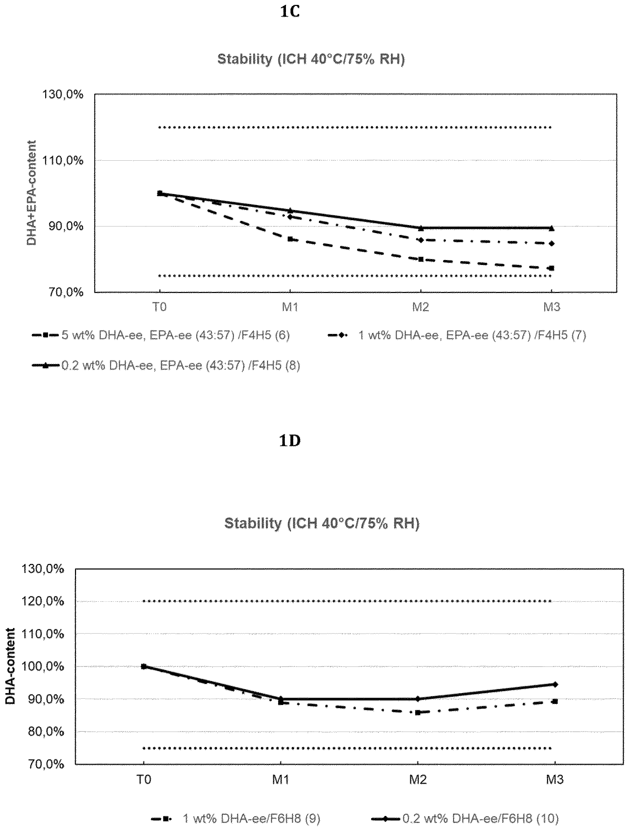

**Figure 1:** Figures 1A-1D are graphical depictions of the stability, based on the content of omega-3 fatty acid ethyl ester, of compositions **1-10** comprising omega-3 fatty acid ethyl ester(s), light liquid paraffin, and a semifluorinated alkane (F4H5 or F6H8) stored in glass or polypropylene vials over a period of 3 months at 40°C at 75% humidity (ICH).

### SUMMARY OF THE INVENTION

The invention relates in a first aspect to a liquid pharmaceutical composition comprising:
a) at least one omega-3 fatty acid ethyl ester in a total amount of up to 5 wt% based on the total weight of the composition;
b) a liquid paraffin in a total amount of up to 1 wt% based on the total weight of the composition;
wherein the at least one omega-3 fatty acid ethyl ester and the liquid paraffin are dissolved in a liquid vehicle comprising a semifluorinated alkane selected from 1-perfluorohexyl-octane (F6H8) or 1-perfluorobutyl-pentane (F4H5), wherein the liquid paraffin is light liquid paraffin.

The liquid vehicle is preferably substantially free of water. In a preferred embodiment the composition is substantially free of water. In a particularly preferred embodiment, the liquid vehicle consists of a semifluorinated alkane.

In a further aspect the invention relates to such a composition for use as a medicament, in particular for topical administration to the eye.

In a further aspect the invention relates to a method for stabilizing such a composition comprising one or more omega-3 fatty acid ethyl esters comprising a step of mixing the one or more omega-3 fatty acid ethyl esters, and optionally one or more antioxidants, with a liquid paraffin and a semifluorinated alkane to form a stable clear and colorless solution; wherein the total amount of liquid paraffin based on the weight of the composition does not exceed the total amount of omega-3 fatty acid ethyl ester based on the total weight of the composition;
wherein the composition retains more than 75 wt% of omega-3 ethyl ester upon storage for 3 months at 40°C at a relative humidity of 75% in a polypropylene or glass container.

### DETAILED DESCRIPTION OF THE INVENTION

In a first aspect, the present invention provides a stable liquid pharmaceutical composition comprising:
a) at least one omega-3 fatty acid ethyl ester in a total amount of up to 5 wt% based on the total weight of the composition;
b) a liquid paraffin in a total amount of up to 1 wt% based on the total weight of the composition;
wherein the at least one omega-3 fatty acid ethyl ester and the liquid paraffin are dissolved in a liquid vehicle comprising a semifluorinated alkane selected from 1-perfluorohexyl-octane (F6H8) or 1-perfluorobutyl-pentane (F4H5), wherein the liquid paraffin is light liquid paraffin.

The inventors found that a liquid composition comprising an omega-3 fatty acid ethyl ester in a liquid vehicle comprising a semifluorinated alkane may be stored over longer periods of time, with a reduced loss of the omega-3 fatty acid ethyl ester active ingredient due to adsorption at the wall of the container, by the inclusion of a liquid paraffin, in a total amount of up to 1 wt % based on the total weight of the composition.

The inventors surprisingly found that the addition of liquid paraffin further improves the storage stability of semifluorinated alkane/omega-3 fatty acid compositions, independently of the presence of an antioxidant

The inventors surprisingly found that a composition as defined above retains more than 75 wt% of omega-3 ethyl ester upon storage for 3 months at 40°c at a relative humidity of 75%. Accordingly, in one embodiment, the invention relates to a composition as defined above, wherein the composition retains more than 75 wt% of omega-3 ethyl ester upon storage for 3 months at 40°C at a relative humidity of 75% in a container. In a preferred embodiment the composition retains more than 95 wt% of omega-3 ethyl ester upon storage for 3 months at 40°C at a relative humidity of 75% in a container. Said container is preferably a glass container or a polypropylene container. In one preferred embodiment, the liquid vehicle is substantially free of water. In a more preferred embodiment, the liquid composition is substantially free of water. In the context of the present invention, substantially free of water refers means that no additional water has been added and any water in the composition may be traces of water associated by the compounds, which are not removed by drying processes.

In a preferred embodiment of the invention, the liquid vehicle substantially consists of the semifluorinated alkane. The liquid vehicle is 1- perfluorohexyl-octane (F6H8) or 1-perfluorobutyl-pentane (F4H5).

The liquid vehicle of the pharmaceutical composition of the present invention comprises or consists of a semifluorinated alkane of the general formula

CF₃(CF₂)ₙ(CH₂)ₘCH₃

wherein n is 5 and m is 7, or wherein n is 3 and m is 4: 1-perfluorohexyl-octane (F6H8) or 1-perfluorobutyl-pentane (F4H5). In a preferred embodiment of the invention the liquid vehicle consists of a semifluorinated alkane selected from F4H5, F6H8 and mixtures thereof.

In a preferred embodiment, the liquid vehicle comprises a semifluorinated alkane wherein the semifluorinated alkane is 1-perfluorohexyl-octane (F6H8). In a further preferred embodiment, the liquid vehicle consists of a semifluorinated alkane wherein the semifluorinated alkane is 1-perfluorohexyl-octane (F6H8).

In yet a further embodiment the liquid vehicle comprises a semifluorinated alkane, wherein the semifluorinated alkane is 1-perfluorobutyl-pentane (F4H5). In a further preferred embodiment, the liquid vehicle consists of a semifluorinated alkane wherein the semifluorinated alkane is 1-perfluorobutyl-pentane (F4H5).

In a particular embodiment, the invention relates to a composition as defined above, wherein the liquid vehicle comprises a semifluorinated alkane or a mixture of semifluorinated alkanes and the semifluorinated alkane or mixtures thereof are present in a total amount of about 93 to 99.6 wt%.

In a specific embodiment the invention relates to a stable liquid pharmaceutical composition comprising:
a) at least one omega-3 fatty acid ethyl ester in a total amount of up to 5 wt% based on the total weight of the composition;
b) a liquid paraffin in a total amount of up to 1 wt% based on the total weight of the composition;
wherein the at least one omega-3 fatty acid ethyl ester and the liquid paraffin are dissolved in a liquid vehicle comprising a semifluorinated alkane selected from 1-perfluorohexyl-octane (F6H8) or 1-perfluorobutyl-pentane (F4H5), wherein the liquid paraffin is light liquid paraffin.

The term wt% (or "% (w/w)"; weight percent) in the context of the present invention refers to the relative amount of a compound, based on the weight of the compound compared to the total weight of the composition.

The deposition of omega-free fatty acid ethyl ester at the gas or air to liquid composition interface, in the form of solid deposits i.e. tidemarks, leading to loss of active omega-3 fatty acid ethyl ester may be reduced, and prevented by the addition of a liquid paraffin to the composition.

The paraffin (also known as paraffinum liquidum) used in the context of the invention may, for example, be a very highly refined mineral oil and/or is based on a mixture of predominantly unsaturated hydrocarbon such as alkanes. As defined herein, the term liquid paraffin, refers to paraffin which is a liquid at ambient (i.e. room temperature) conditions.

The addition of the liquid paraffin, in a defined amount according to the invention, does not affect the refractive index of the composition. The liquid paraffin featured in the context of the present invention should also be biocompatible, i.e. physiologically acceptable and tolerable for administration to the eye and tissues of the eye.

The inventors have found, in particular, that liquid paraffin is particularly suitable, and does not have a negative impact, with no or only marginal changes in the refractive index of the composition. Accordingly, there is minimal effect on vision (e.g. no blurriness) for topical application to the eye.

The total amount of the liquid paraffin featured in the composition according to the invention is up 1 wt%, or less. In a preferred embodiment of the invention, the total amount of paraffin is up to about 0.5 wt% or less. In a particular embodiment of the invention, the total amount of paraffin in the composition is up to about 0.2 wt% or less. In a further embodiment of the invention, the total amount of paraffin is up to about 0.1 wt% or less.

In some embodiments the total amount of liquid paraffin featured in the composition is between about 0.1 wt% and 1 wt%. In a further embodiment of the invention, the total amount of paraffin in the composition is between about 0.1 wt% and about 0.5 wt%. In a particular embodiment of the invention, the total amount of paraffin in the composition is between about 0.1 wt% and about 0.2 wt%.

It has been found that the deposition of the omega-3 fatty acid ethyl ester in the form of tidemarks in the containers over time during storage may be reduced, in particular where the relative ratio of the amount of paraffin in the composition compared to the amount of the omega-3 fatty acid ethyl ester based on their weight is similar. In one embodiment, the relative ratio of the amount of paraffin in the composition compared to the amount of the omega-3 fatty acid ethyl ester based on their weight is between 2:1 and 1:25.

In one embodiment, the total amount of liquid paraffin does not exceed the total amount of omega-3 fatty acid esters. In a further embodiment, the relative ratio of the amount of paraffin to the amount of the at least one omega-3 fatty acid ethyl ester is between 1:1 and 1:5. In another embodiment of the invention, the relative ratio of the amount of paraffin in the composition compared to the amount of the omega-3 fatty acid ethyl ester based on their weight is about 1:1.

The at least one omega-3 fatty acid ethyl ester according to the invention may be any omega-3 fatty acid ethyl ester acceptable for pharmaceutical use, in particular acceptable for use in ophthalmic applications.

In a preferred embodiment, the at least one omega-3 fatty acid ethyl ester is selected from the group consisting of eicosapentaenoic acid ethyl ester (EPA-ee), docosahexaenoic acid ethyl ester (DHA-ee), alpha-linolenic acid ethyl ester (ALA-ee) and mixtures thereof.

In one embodiment, the omega-3-fatty acid ethyl ester is selected from eicosapentaenoic acid ethyl ester (EPA-ee), docosahexaenoic acid ethyl ester (DHA-ee) and a mixture thereof, which are particularly suitable for ophthalmic application, e.g. in the treatment of keratoconjunctivitis sicca, Meibomian Gland Dysfunction or a symptom associated therewith.

Accordingly, in a particular preferred embodiment of the invention, the at least one omega 3 fatty acid ethyl ester is eicosapentaenoic acid ethyl ester, docosahexaenoic ethyl ester or a mixture thereof.

The at least one omega-3 fatty acid ethyl ester can also be a mixture of omega-3 fatty acid ethyl esters.

In one embodiment of the invention, the at least one omega 3 fatty acid ethyl ester is a mixture of eicosapentaenoic acid ethyl ester and docosahexaenoic acid ethyl ester. In a preferred embodiment of the invention the at least one omega 3 fatty acid ethyl ester is a mixture of eicosapentaenoic acid ethyl ester and docosahexaenoic acid ethyl ester at a ratio of about 1:5 to 5:1, preferably 2:1 to 1:2, more preferably at a ratio of about 1:1 (e.g. a mixture of EPA-ee to DHA-ee at a ratio of 57:43) .

Preferably, the combined amount of all omega-3 fatty acid ethyl esters does not exceed 5 wt% of the total weight of the composition, preferably the combined amount of all omega-3 fatty acid ethyl esters does not exceed 2.5 wt% of the total weight of the composition.

The omega-3 fatty acid ethyl ester may be obtained from any suitable source. Common sources for omega-3 fatty acid esters include fish oils, for example tuna oil or cod liver oil, or plant oils, in particular plant seed oils, such as linseed oil or black raspberry oil.

In a preferred embodiment of the invention, the at least one omega-3 ethyl ester is of fish, algae, plant or vegan origin. In a particular preferred embodiment, the at least one omega-3 fatty acid ethyl ester is of algae origin.

If the at least one omega-3 fatty acid ethyl ester is a mixture of esters, these may be from the same source or different sources. In a preferred embodiment of the invention, the at least one omega-3 fatty acid ethyl ester is a mixture of omega-3 fatty acid ethyl esters, wherein the omega-3 fatty acid ethyl esters originate from the same source. Preferably, said omega-3 fatty acid ethyl esters are from the same source of fish, algae, plant or vegan origin, preferably algae origin.

Independent of whether the at least one omega-3 fatty acid ethyl ester in the composition is a single ester or a mixture of ester compounds, it is preferred that the total amount of liquid paraffin in wt% does not exceed the total amount of omega-3 fatty acid ethyl ester in wt%. In a preferred embodiment of the invention, the relative ratio of the amount of paraffin based on the total weight of the composition to the amount of the at least one omega-3 fatty acid ethyl ester based on the total weight of the composition is no more than about 1:1, or about 1:1.

The total amount of omega-3 fatty acid ethyl ester in the composition in one embodiment is 5 wt% or less. In some embodiments the total amount of omega-3 fatty acid ethyl ester is 2.5 wt%. In some embodiments of the invention, the total amount of omega 3 fatty acid ethyl ester is 2 wt% or less, preferably 1 %wt or less, particularly preferably 0.5 wt% or less.

In a particular preferred embodiment of the invention, the total amount of the at least one omega-3 fatty acid ethyl ester is up to about 0.2 wt% based on the total weight of the composition.

In some embodiments, the total amount of omega-3 fatty acid ethyl ester in the composition is between about 0.1 wt% and 5 wt%. In some embodiments, the total amount of omega-3 fatty acid ethyl ester in the composition is between about 0.1 wt% and about 2.5 wt%. In some embodiments of the invention, the total amount of omega 3 fatty acid ethyl ester is between about 0.1 wt% and about 2 wt%, preferably between about 0.1 wt% and about 1 %wt, particularly preferably between about 0.1 wt% and about 0.5 wt%.

In a particular preferred embodiment of the invention, the total amount of the at least one omega-3 fatty acid ethyl ester is between about 0.1 wt% and about 0.2 wt% based on the total weight of the composition.

The composition may additionally comprise further pharmaceutically acceptable excipients and/or additives. In particular, the pharmaceutical composition may comprise antioxidants, preservatives, odor and/or taste masking agents.

In a particular embodiment, the invention relates to a liquid pharmaceutical composition, additionally comprising one or more antioxidants. Any pharmaceutically acceptable antioxidant may be used in the composition. Preferably, antioxidants are selected from the group consisting of alpha-tocopherol, butylhydroxytoluene, butylhydroxyanisole, ascorbyl palmitate and mixtures thereof.

In a particular preferred embodiment, the invention relates to a liquid pharmaceutical composition as defined above, additionally comprising one or more antioxidants, wherein the one or more antioxidants are selected from group consisting of alpha-tocopherol, butylhydroxytoluene, butylhydroxyanisole, ascorbyl palmitate and mixtures thereof. The antioxidant is also dissolved in the liquid vehicle comprising or consisting of said semifluorinated alkane

In general, the one or more antioxidants should be present in relatively low amounts. In particular, the one or more antioxidants are present in a total amount of up to 0.1 wt% or less based on the total weight of the composition. In a preferred embodiment of the invention, the one or more antioxidants are present in a total amount of up to 0.05 wt% based on the total weight of the composition. In a particular preferred embodiment, the one or more antioxidants are present in a total amount of up to 0.02 wt% based on the total weight of the composition.

In some embodiments, the one or more antioxidants are present in a total amount between about 0.001 wt% and about 0.1 wt% based on the total weight of the composition. In a preferred embodiment of the invention, the one or more antioxidants are present in a total amount of about 0.001 wt% and about 0.05 wt% based on the total weight of the composition. In a particular preferred embodiment, the one or more antioxidants are present in a total amount of about 0.001 wt% and about 0.02 wt% based on the total weight of the composition.

While the composition may comprise additional compounds, in an embodiment of the invention, the composition may be free of any further excipients or additives aside from one or more antioxidants. In particular, it is preferred that the composition is substantially free of water.

In addition, the composition may be free of a preservative, such as an anti-microbial agent. It is further preferred that the composition is free of taste and/or odor masking agents. It is particularly preferred, that the composition is sterile.

Accordingly, in a particular embodiment, the invention relates to a composition as defined above, wherein the composition is sterile, substantially free of water and/or free of a preservative. In a specific embodiment, the invention relates to a composition as defined above, wherein the composition is free of a taste-masking agent or odor masking agent.

According to a specific embodiment, the invention relates to a composition as defined above, that is odorless, more preferably the composition comprises an omega-3 fatty acid ethyl ester of algae origin and is odorless.

In a specific embodiment, the invention relates to a composition as defined above, wherein the composition is sterile, substantially free of water, free of a preservative, and/or free of a taste-masking agent and/or odor masking agent

In a further specific embodiment, the invention relates to a liquid pharmaceutical composition comprising
up to 5 wt% of at least one omega-3 fatty acid ethyl ester;
up to 1 wt% of liquid paraffin;
dissolved in a liquid vehicle comprising a semifluorinated alkane selected from 1-perfluorohexyloctane (F6H8), 1-perfluorobutylpentane (F4H5) or mixtures thereof.
wherein wt% is based on the total weight of the composition; wherein the total amount of paraffin in wt% does not exceed the total amount of omega-3 fatty acid ester wherein the liquid paraffin is light liquid paraffin.

In a further specific embodiment, the invention relates to a liquid pharmaceutical composition comprising
up to 1 wt% of at least one omega-3 fatty acid ethyl ester;
up to 1 wt% of liquid paraffin;
dissolved in a liquid vehicle comprising a semifluorinated alkane selected from 1-perfluorohexyloctane (F6H8), 1-perfluorobutylpentane (F4H5) or mixtures thereof.
wherein wt% is based on the total weight of the composition; wherein the total amount of paraffin in wt% does not exceed the total amount of omega-3 fatty acid ester, wherein the liquid paraffin is light liquid paraffin.

In a yet further specific embodiment, the invention relates to a liquid pharmaceutical composition comprising
up to 0.2 wt% of at least one omega-3 fatty acid ethyl ester;
up to 0.2 wt% of liquid paraffin;
dissolved in a liquid vehicle comprising a semifluorinated alkane selected from 1-perfluorohexyloctane (F6H8), 1-perfluorobutylpentane (F4H5) or mixtures thereof.
wherein wt% is based on the total weight of the composition; wherein the total amount of paraffin in wt% does not exceed the total amount of omega-3 fatty acid ester, wherein the liquid paraffin is light liquid paraffin.

In another embodiment, the composition according to the invention comprises
up to 5 wt% of at least one omega-3 fatty acid ester selected from docosahexaenoic acid ethyl ester, eicosapentaenoic acid ethyl ester and mixtures thereof;
up to 1 wt% of light liquid paraffin;
dissolved in a liquid vehicle comprising a semifluorinated alkane selected from 1-perfluorohexyloctane (F6H8), 1-perfluorobutylpentane (F4H5) or mixtures thereof.
wherein wt% is based on the total weight of the composition; wherein the total amount of light liquid paraffin in wt% does not exceed the total amount of omega-3 fatty acid ester.

In another embodiment, the composition according to the invention comprises
up to 1 wt% of at least one omega-3 fatty acid ester selected from docosahexaenoic acid ethyl ester, eicosapentaenoic acid ethyl ester and mixtures thereof;
up to 1 wt% of light liquid paraffin;
dissolved in a liquid vehicle comprising a semifluorinated alkane selected from 1-perfluorohexyloctane (F6H8), 1-perfluorobutylpentane (F4H5) or mixtures thereof.
wherein wt% is based on the total weight of the composition; wherein the total amount of light liquid paraffin in wt% does not exceed the total amount of omega-3 fatty acid ester.

In another embodiment, the composition according to the invention comprises
up to 0.2 wt% of at least one omega-3 fatty acid ester selected from docosahexaenoic acid ethyl ester, eicosapentaenoic acid ethyl ester and mixtures thereof;
up to 0.2 wt% of light liquid paraffin;
dissolved in a liquid vehicle comprising a semifluorinated alkane selected from 1-perfluorohexyloctane (F6H8), 1-perfluorobutylpentane (F4H5) or mixtures thereof.
wherein wt% is based on the total weight of the composition; wherein the total amount of light liquid paraffin in wt% does not exceed the total amount of omega-3 fatty acid ester.

In another embodiment, the composition according to the invention comprises 0.2 wt% docosahexaenoic acid ethyl ester, 0.2 wt% light liquid paraffin dissolved in F6H8; wherein wt% is based on the total weight of the composition.

In a specific embodiment, the invention relates to a liquid pharmaceutical composition comprising
up to 5 wt% of at least one omega-3 fatty acid ethyl ester;
up to 1 wt% of liquid paraffin;
optionally up to 0.1 wt% of antioxidant;
wherein the at least one omega-3 fatty acid ethyl ester, the liquid paraffin and the optional antioxidant are dissolved in a liquid vehicle comprising a semifluorinated alkane selected from 1-perfluorohexyloctane (F6H8), 1-perfluorobutylpentane (F4H5) or mixtures thereof. wherein wt% is based on the total weight of the composition; wherein the total amount of paraffin in wt% does not exceed the total amount of omega-3 fatty acid ester, wherein the liquid paraffin is light liquid paraffin.

In a particular embodiment, the invention relates to a liquid pharmaceutical composition comprising
up to 1 wt% of at least one omega-3 fatty acid ester selected from docosahexaenoic acid ethyl ester, eicosapentaenoic acid ethyl ester and mixtures thereof;
up to 1 wt% of liquid paraffin;
optionally up to 0.1 wt% of antioxidant;
wherein the at least one omega-3 fatty acid ethyl ester, the liquid paraffin and the optional antioxidant are dissolved in a liquid vehicle comprising a semifluorinated alkane selected from 1-perfluorohexyloctane (F6H8), 1-perfluorobutylpentane (F4H5) or mixtures thereof., wherein wt% is based on the total weight of the composition; wherein the total amount of paraffin in wt% does not exceed the total amount of omega-3 fatty acid ester, wherein the liquid paraffin is light liquid paraffin.

In a further particular embodiment, the invention relates to a liquid pharmaceutical composition comprising
up to 0.2 wt% of at least one omega-3 fatty acid ester selected from docosahexaenoic acid ethyl ester, eicosapentaenoic acid ethyl ester and mixtures thereof;
up to 0.2 wt% of liquid paraffin;
optionally up to 0.1 wt% of antioxidant;
wherein the at least one omega-3 fatty acid ethyl ester, the liquid paraffin and the optional antioxidant are dissolved in a liquid vehicle comprising a semifluorinated alkane selected from 1-perfluorohexyloctane (F6H8), 1-perfluorobutylpentane (F4H5) or mixtures thereof, wherein wt% is based on the total weight of the composition; wherein the total amount of paraffin in wt% does not exceed the total amount of omega-3 fatty acid ester, wherein the liquid paraffin is light liquid paraffin.

In a specific embodiment, the invention relates to a liquid pharmaceutical composition comprising
up to 1 wt% of at least one omega-3 fatty acid ester selected from docosahexaenoic acid ethyl ester, eicosapentaenoic acid ethyl ester and mixtures thereof;;
up to 1 wt% of liquid paraffin;
optionally up to 0.1 wt% of antioxidant;
wherein the at least one omega-3 fatty acid ethyl ester, the liquid paraffin and the optional antioxidant are dissolved in a liquid vehicle comprising a semifluorinated alkane selected from 1-perfluorohexyl-octane (F6H8) or 1-perfluorobutyl-pentane (F4H5),
wherein wt% is based on the total weight of the composition; wherein the total amount of paraffin in wt% does not exceed the total amount of omega-3 fatty acid ester, wherein the liquid paraffin is light liquid paraffin.

In a particular embodiment of the invention, the invention relates to a composition essentially consisting of up to 5 wt% of an omega-3 fatty acid ethyl ester selected from docosahexaenoic acid ethyl ester, eicosapentaenoic acid ethyl ester or mixtures thereof, 0.2 wt% light liquid paraffin dissolved in a semifluorinated alkane selected from F4H5, F6H8 and mixtures thereof and optionally up to 0.1 wt% of antioxidant

In a particular embodiment of the invention, the invention relates to a composition essentially consisting of up to 1 wt% of an omega-3 fatty acid ethyl ester selected from docosahexaenoic acid ethyl ester, eicosapentaenoic acid ethyl ester or mixtures thereof, 0.2 wt% light liquid paraffin dissolved in a semifluorinated alkane selected from F4H5, F6H8 and mixtures thereof and optionally up to 0.1 wt% of antioxidant

In a particular embodiment of the invention, the invention relates to a composition essentially consisting of up to 0.2 wt% of an omega-3 fatty acid ethyl ester selected from docosahexaenoic acid ethyl ester, eicosapentaenoic acid ethyl ester or mixtures thereof, 0.2 wt% light liquid paraffin dissolved in a semifluorinated alkane selected from F4H5, F6H8 and mixtures thereof and optionally up to 0.1 wt% of antioxidant

In a particular embodiment of the invention, the invention relates to a composition as defined above, wherein the composition retains more than 95 wt% of omega-3 ethyl ester upon storage for 3 months at 40°C at a relative humidity of 75% in a glass container.

In an alternative embodiment of the invention, the invention relates to a composition as defined above, wherein the composition retains more than 75 wt% of omega-3 ethyl ester upon storage for 3 months at 40°C at a relative humidity of 75% in a polypropylene container

In a particular embodiment, the invention relates to a liquid pharmaceutical composition comprising
up to 1 wt% of at least one omega-3 fatty acid ethyl ester, selected from the group consisting of eicosapentaenoic acid ethyl ester (EPA-ee), docosahexaenoic acid ethyl ester(DHA-ee), alpha-linolenic acid ethyl ester (ALA-ee) and mixtures thereof;
up to 1 wt% of light liquid paraffin;
optionally up to 0.1 wt% of antioxidant, selected from the group consisting of alpha-tocopherol, butylhydroxytoluene, butylhydroxyanisole, ascorbyl palmitate and mixtures thereof;
wherein the at least one omega-3 fatty acid ethyl ester, the liquid paraffin and the optional antioxidant are dissolved in a liquid vehicle comprising a semifluorinated alkane, more preferably consisting of a semifluorinated alkane, selected from 1-perfluorohexyl-octane (F6H8) or 1-perfluorobutyl-pentane (F4H5) or mixtures thereof.
wherein wt% is based on the total weight of the composition; wherein the total amount of paraffin in wt% does not exceed the total amount of omega-3 fatty acid ester.

In a particular embodiment, the invention relates to a liquid pharmaceutical composition comprising
up to 0.2 wt% of at least one omega-3 fatty acid ethyl ester, selected from the group consisting of eicosapentaenoic acid ethyl ester (EPA-ee), docosahexaenoic acid ethyl ester(DHA-ee), alpha-linolenic acid ethyl ester (ALA-ee) and mixtures thereof;
up to 0.2 wt% of light liquid paraffin;
optionally up to 0.1 wt% of antioxidant, selected from the group consisting of alpha-tocopherol, butylhydroxytoluene, butylhydroxyanisole, ascorbyl palmitate and mixtures thereof;
wherein the at least one omega-3 fatty acid ethyl ester, the liquid paraffin and the optional antioxidant are dissolved in a liquid vehicle comprising a semifluorinated alkane, more preferably consisting of a semifluorinated alkane, selected from 1-perfluorohexyl-octane (F6H8) or 1-perfluorobutyl-pentane (F4H5) or mixtures thereof.
wherein wt% is based on the total weight of the composition; wherein the total amount of paraffin in wt% does not exceed the total amount of omega-3 fatty acid ester.

In particular embodiments, the liquid composition merely comprises or consists of a minimum of compounds. Preferably, the composition consists only of an omega-3 fatty acid ethyl ester or a mixture of omega-3 fatty acid ethyl esters, light liquid paraffin and optionally an antioxidant dissolved in a liquid vehicle comprising a semifluorinated alkane selected from 1-perfluorohexyl-octane (F6H8) or 1-perfluorobutyl-pentane (F4H5).

In a particular preferred embodiment, the invention relates to a composition as defined above, wherein the composition substantially consists of at least one omega-3 fatty acid ethyl ester, a liquid paraffin and optionally an antioxidant dissolved in a liquid vehicle comprising a semifluorinated alkane. More preferably, the invention relates to a composition as defined above, wherein the composition substantially consists of at least one omega-3 fatty acid ethyl ester, a liquid paraffin and optionally an antioxidant dissolved in a semifluorinated alkane.

The semifluorinated alkane is selected from the group consisting of 1-perfluorohexyl-octane (F6H8) and 1-perfluorobutyl-pentane (F4H5). In some embodiments, the semifluorinated alkane is 1-perfluorohexyl-octane (F6H8), and in some embodiments the semifluorinated alkane is 1-perfluorobutyl-pentane (F4H5).

In specific embodiments the invention relates to a stable liquid pharmaceutical composition consisting of:
a) at least one omega-3 fatty acid ethyl ester in a total amount of up to 1 wt% based on the total weight of the composition;
b) a liquid paraffin in a total amount of up to 1 wt% based on the total weight of the composition;
c) optionally, an antioxidant in a total amount of up to 0.1 wt% or less based on the total weight of the composition,
wherein the at least one omega-3 fatty acid ethyl ester, the liquid paraffin and the optional antioxidant are dissolved in a liquid vehicle comprising a semifluorinated alkane, more preferably consisting of a semifluorinated alkane, selected from 1-perfluorohexyl-octane (F6H8) or 1-perfluorobutyl-pentane (F4H5), wherein the liquid paraffin is light liquid paraffin.

In further specific embodiments the invention relates to a stable liquid pharmaceutical composition consisting of:
a) at least one omega-3 fatty acid ethyl ester in a total amount of up to 0.2 wt% based on the total weight of the composition;
b) a liquid paraffin in a total amount of up to 0.2 wt% based on the total weight of the composition;
c) optionally, an antioxidant in a total amount of up to 0.1 wt% or less based on the total weight of the composition,
wherein the at least one omega-3 fatty acid ethyl ester, the liquid paraffin and the optional antioxidant are dissolved in a liquid vehicle comprising a semifluorinated alkane, more preferably consisting of a semifluorinated alkane, selected from 1-perfluorohexyl-octane (F6H8) or 1-perfluorobutyl-pentane (F4H5), wherein the liquid paraffin is light liquid paraffin.

The present invention relates to in particular, but is not limited to, the following compositions:
In one embodiment the invention relates to a liquid composition comprising
up to 1 wt% docosahexaenoic acid ethyl ester and/or up to 1 wt% eicosapentaenoic acid ethyl ester;
up to 1 wt% of light liquid paraffin;
up to 0.1 wt% of antioxidant;
wherein the at least one omega-3 fatty acid ethyl ester, the liquid paraffin and the optional antioxidant are dissolved in a liquid vehicle comprising a semifluorinated alkane selected from F4H5 and F6H8, preferably 1-perfluorohexyl-octane (F6H8);
wherein wt% is based on the total weight of the composition; wherein the total amount of light liquid paraffin in wt% does not exceed the total amount of omega-3 fatty acid ester.

In a particular embodiment, the invention relates to a composition essentially consisting of up to 1 wt% of an omega-3 fatty acid ethyl ester selected from docosahexaenoic acid ethyl ester, eicosapentaenoic acid ethyl ester or mixtures thereof, 0.2 wt% light liquid paraffin dissolved in a semifluorinated alkane selected from F4H5, F6H8 and mixtures thereof and optionally up to 0.1 wt% of antioxidant

In a particular embodiment, the invention relates to a composition essentially consisting of up to 0.2 wt% of an omega-3 fatty acid ethyl ester selected from docosahexaenoic acid ethyl ester, eicosapentaenoic acid ethyl ester or mixtures thereof, 0.2 wt% light liquid paraffin dissolved in a semifluorinated alkane selected from F4H5, F6H8 and mixtures thereof and optionally up to 0.1 wt% of antioxidant

In a particular embodiment, the invention relates to a liquid composition comprising
up to 0.5 wt% docosahexaenoic acid ethyl ester and/or up to 0.5 wt% eicosapentaenoic acid ethyl ester;
up to 0.5 wt% of light liquid paraffin;
up to 0.1 wt% of antioxidant;
wherein the at least one omega-3 fatty acid ethyl ester, the liquid paraffin and the optional antioxidant are dissolved in a liquid vehicle comprising a semifluorinated alkane selected from F4H5 and F6H8" preferably 1-perfluorohexyl-octane (F6H8);
wherein wt% is based on the total weight of the composition; wherein the total amount of light liquid paraffin in wt% does not exceed the total amount of omega-3 fatty acid ester.

In a further embodiment the invention relates to a liquid composition comprising
up to 1 wt% of an omega-3 fatty acid ethyl ester selected from docosahexaenoic acid ethyl ester, eicosapentaenoic acid ethyl ester or mixtures thereof;
up to 0.2 wt% of light liquid paraffin;
optionally up to 0.1 wt% of antioxidant;
at least 98 wt% of a semifluorinated alkane selected from F4H5, F6H8 and mixtures thereof wherein wt% is based on the total weight of the composition; wherein the total amount of light liquid paraffin in wt% does not exceed the total amount of omega-3 fatty acid ester.

In a further embodiment the invention relates to a liquid composition comprising
up to 0.2 wt% of an omega-3 fatty acid ethyl ester selected from docosahexaenoic acid ethyl ester, eicosapentaenoic acid ethyl ester or mixtures thereof;
up to 0.2 wt% of light liquid paraffin;
optionally up to 0.1 wt% of antioxidant;
at least 99 wt% of a semifluorinated alkane selected from F4H5, F6H8 and mixtures thereof wherein wt% is based on the total weight of the composition; wherein the total amount of light liquid paraffin in wt% does not exceed the total amount of omega-3 fatty acid ester.

In a further embodiment the invention relates to a liquid composition comprising
up to 0.1 wt% docosahexaenoic acid ethyl ester and 0.1 wt% eicosapentaenoic acid ethyl ester;
up to 0.2 wt% of light liquid paraffin;
up to 0.1 wt% of antioxidant;
wherein the at least one omega-3 fatty acid ethyl ester, the liquid paraffin and antioxidant are dissolved in a liquid vehicle comprising a semifluorinated alkane selected from F4H5, F6H8 and mixtures thereof,, preferably 1-perfluorohexyl-octane (F6H8);
wherein wt% is based on the total weight of the composition; wherein the total amount of light liquid paraffin in wt% does not exceed the total amount of omega-3 fatty acid ester.

In a further embodiment, the invention relates to a composition as defined above, comprising up to 1 wt% docosahexaenoic acid ethyl ester, 0.2 wt% light liquid paraffin, at least 98 wt% of a semifluorinated alkane selected from F4H5, F6H8 and mixtures thereof and optionally up to 0.1 wt% of antioxidant.

In a further embodiment, the invention relates to a composition as defined above, essentially consisting of 1 wt% docosahexaenoic acid ethyl ester, 0.2 wt% light liquid paraffin, dissolved in a semifluorinated alkane selected from F4H5, F6H8 and mixtures thereof and optionally up to 0.1 wt% of antioxidant

In another specific embodiment, the invention relates to a composition as defined above, essentially consisting of 1 wt% docosahexaenoic acid ethyl ester, 0.2 wt% light liquid paraffin, optionally 0.0133 wt% butylhydroxyanisole, and/or optionally 0.0067 wt% butylhydroxytoluene dissolved in F6H8.

In another specific embodiment, the invention relates to a composition as defined above, essentially consisting of 1 wt% docosahexaenoic acid ethyl ester, 0.2 wt% light liquid paraffin, 0.0133 wt% butylhydroxyanisole, and 0.0067 wt% butylhydroxytoluene dissolved in F6H8.

In a further embodiment, the invention relates to a composition as defined above, comprising up to 0.2 wt% docosahexaenoic acid ethyl ester, 0.2 wt% light liquid paraffin, at least 99 wt% of a semifluorinated alkane selected from F4H5, F6H8 and mixtures thereof and optionally up to 0.1 wt% of antioxidant.

In a further embodiment, the invention relates to a composition as defined above, essentially consisting of 0.2 wt% docosahexaenoic acid ethyl ester, 0.2 wt% light liquid paraffin, dissolved in a semifluorinated alkane selected from F4H5, F6H8 and mixtures thereof and optionally up to 0.1 wt% of antioxidant

In a further specific embodiment, the invention relates to a composition as defined above, comprising 0.2 wt% docosahexaenoic acid ethyl ester, 0.2 wt% light liquid paraffin, optionally 0.0133 wt% butylhydroxyanisole, and/or optionally 0.0067 wt% butylhydroxytoluene dissolved in F6H8.

In a further specific embodiment, the invention relates to a composition as defined above, essentially consisting of 0.2 wt% docosahexaenoic acid ethyl ester, 0.2 wt% light liquid paraffin, 0.0133 wt% butylhydroxyanisole, and 0.0067 wt% butylhydroxytoluene dissolved in F6H8.

In a different specific embodiment, the invention relates to a composition as defined above, comprising up to 1 wt% eicosapentaenoic acid ethyl ester, 0.2 wt% light liquid paraffin, at least 98 wt% of a semifluorinated alkane selected from F4H5, F6H8 and mixtures thereof and optionally up to 0.1 wt% of antioxidant

In a different specific embodiment, the invention relates to a composition as defined above, essentially consisting of 1 wt% eicosapentaenoic acid ethyl ester, 0.2 wt% light liquid paraffin, dissolved in a semifluorinated alkane selected from F4H5, F6H8 and mixtures thereof and optionally up to 0.1 wt% of antioxidant

In another specific embodiment, the invention relates to a composition as defined above, essentially consisting of 1 wt% eicosapentaenoic acid ethyl ester, 0.2 wt% light liquid paraffin, optionally 0.0133 wt% butylhydroxyanisole, and/or optionally 0.0067 wt% butylhydroxytoluene dissolved in F6H8.

In a different specific embodiment, the invention relates to a composition as defined above, comprising up to 0.2 wt% eicosapentaenoic acid ethyl ester, 0.2 wt% light liquid paraffin, at least 99 wt% of a semifluorinated alkane selected from F4H5, F6H8 and mixtures thereof and optionally up to 0.1 wt% of antioxidant

In a different specific embodiment, the invention relates to a composition as defined above, essentially consisting of 0.2 wt% eicosapentaenoic acid ethyl ester, 0.2 wt% light liquid paraffin, dissolved in a semifluorinated alkane selected from F4H5, F6H8 and mixtures thereof and optionally up to 0.1 wt% of antioxidant

In another specific embodiment, the invention relates to a composition as defined above, essentially consisting of 0.2 wt% eicosapentaenoic acid ethyl ester, 0.2 wt% light liquid paraffin, optionally 0.0133 wt% butylhydroxyanisole, and/or optionally 0.0067 wt% butylhydroxytoluene dissolved in F6H8.

In a particular specific embodiment, the invention relates to a composition as defined above, comprising 0.1 wt% docosahexaenoic acid ethyl ester, 0.1 wt% eicosapentaenoic acid ethyl ester, 0.2 wt% light liquid paraffin, optionally 0.0133 wt% butylhydroxyanisole, and/or 0.0067 wt% butylhydroxytoluene dissolved in F6H8.

In one embodiment, the invention relates to a composition as defined above, essentially consisting of 0.1 wt% docosahexaenoic acid ethyl ester, 0.1 wt% eicosapentaenoic acid ethyl ester, 0.2 wt% light liquid paraffin, optionally 0.0133 wt% butylhydroxyanisole, and/or 0.0067 wt% butylhydroxytoluene dissolved in F6H8.

In a further specific embodiment, the invention relates to a composition as defined above, comprising 0.2 wt% docosahexaenoic acid ethyl ester, 0.2 wt% light liquid paraffin, and optionally 0.0004 wt % alpha-tocopherol dissolved in F6H8.

In one particular embodiment, the invention relates to a composition as defined above, essentially consisting of 0.2 wt% docosahexaenoic acid ethyl ester, 0.2 wt% light liquid paraffin, and optionally 0.0004 wt % alpha-tocopherol dissolved in F6H8.

In a further embodiment, the invention relates to a composition as defined above, comprising 0.2 wt% eicosahexaenoic acid ethyl ester, 0.2 wt% light liquid paraffin, and optionally 0.0004 wt % alpha-tocopherol dissolved in F6H8.

In an additional embodiment, the invention relates to a composition as defined above, essentially consisting of 0.2 wt% eicosahexaenoic acid ethyl ester, 0.2 wt% light liquid paraffin, and optionally 0.0004 wt % alpha-tocopherol dissolved in F6H8.

These specific compositions as defined above may also be dissolved in 1-perfluorobutyl-pentane (F4H5) instead of 1-perfluorohexyl-octane (F6H8). The compositions above are intended to illustrate particular preferred embodiments.

The inventors found that the compositions above retain more than 95 wt% of omega-3 ethyl ester upon storage for 3 months at 40°C at a relative humidity of 75% in a glass container and retain more than 85 wt% of omega-3 ethyl ester upon storage for 3 months at 40°C at a relative humidity of 75% in a polypropylene container.

In a second aspect, the invention relates to the compositions as defined above for medical and pharmaceutical uses.

Accordingly, in one embodiment the invention relates to a composition as defined above for use as a medicament The invention relates in particular to medicaments suitable or formulated for ophthalmic application, especially topical ophthalmic application.

In a particular embodiment, the invention relates to a composition according to any one of the above definitions or embodiments for use as a medicament, wherein the medicament is an ophthalmic administered medicament. Preferably, said medicament is topically administered to the eye, for example directly to the surface of the eye accessible to topical administration or instillation, for example to the cornea or to the conjunctiva. The medicament may also be administered alternatively into a space e.g. a sac or pocket formed by gently pulling down of a lower eyelid.

Depending on the indication, the composition may be administered by other means such as by injection, or to other tissues. However, it is preferred that the composition is topically applied.

The composition according to the present invention is particularly suitable for the treatment or alleviation of keratoconjunctivitis sicca and conditions and symptoms associated therewith.

Keratoconjunctivitis sicca (also known as dry eye disease, or dry eye syndrome) is a complex disease that results in symptoms of discomfort, visual disturbance, and tear film instability, and which creates potential for damage of the ocular surface. It may be accompanied by increased osmolarity of the tear film and inflammation of the ocular surface. A patient having keratoconjunctivitis sicca may experience any one off, or a combination of tear hyperosmolarity, tear film instability or abnormalities in the lipid layer composition of the tear film. Two major categories of keratoconjunctivitis sicca or dry eye disease (DED) may be distinguished, aqueous-deficient dry eye disease and evaporative dry eye disease. In one embodiment of the invention, the composition of the present invention may be used to treat aqueous-deficient dry eye disease and/or evaporative dry eye disease.

Within the class of aqueous-deficient forms of DED, two major subtypes may be differentiated, Sjögren and non-Sjögren. Sjögren syndrome patients suffer from autoimmune disorders in which the lacrimal glands are invaded by activated T-cells, which leads not only to dry eye disease but also to a dry mouth condition. The Sjögren syndrome can be a primary disease or result from other autoimmune diseases such as systemic lupus erythematosus or rheumatoid arthritis. Non-Sjögren patients suffering from an aqueous-deficient DED usually have a lacrimal gland insufficiency, lacrimal duct obstruction or reflex hyposecretion.

The second major class, evaporative dry eye disease, is heterogeneous and can develop as a result of diverse root causes. Major causes include meibomian gland disease or dysfunction, eyelid aperture disorders, blink disorders (as in Parkinson disease) or ocular surface disorders (as in allergic conjunctivitis).

In particular, Meibomian gland diseases and dysfunctions are prevalently associated with evaporative dry eye disease. In one particular embodiment, the composition according to the present invention may also be suitable for the treatment and/or alleviation of meibomian gland dysfunction (also abbreviated MGD). Meibomian gland dysfunction can result in changes in the quantitative or qualitative secretion of the lipid components required for the tear film. The meibum can also have an altered composition, enriched in some components and/or deficient in other components, compared to normal meibum. This may result in altered physical properties, such as abnormal viscosity or abnormal solubility. This in turn can lead to a failure in forming a stable and continuous tear film, which is followed by evaporative loss and hyperosmolarity. Meibomian gland dysfunction can often be characterized by gland obstruction and clogging through hyperkeratinisation of the gland and increased viscosity of the meibum. Dysfunction can arise from a primary lid-margin related disease or a secondary disease arising from systemic disorders such as acne rosacea or seborrheic dermatitis.

Symptoms of keratoconjunctivitis sicca include a dry, scratchy, gritty, or sandy feeling in the eye; foreign body sensation; pain or soreness; stinging or burning; itching; increased blinking; eye fatigue; photophobia; blurry vision; redness; mucus discharge; contact lens intolerance; excessive reflex tearing. In addition to the symptoms of keratoconjunctivitis sicca as described, patients with Meibomian gland dysfunction may also experience symptoms including itchiness, redness, swelling, pain or soreness, discharge accumulation or crusting specifically at the lid margins. It is understood that not all patients suffering from keratoconjunctivitis sicca exhibit all symptoms simultaneously. Hence, there is currently no uniform set of criteria for diagnosing the disease. It is also understood that patients may suffer from one or more subtypes of keratoconjunctivitis sicca, or one or more conditions or disease pathways causing keratoconjunctivitis sicca. It is however important to note that, within the scope of the present invention, any of the aspects, symptoms or pathophysiological consequences of dry eye disease may be addressed.

In one particular embodiment, the invention relates to a composition as defined above for use as a medicament, wherein the medicament is for the prevention or therapy of dry eye disease (keratoconjunctivitis sicca), evaporative dry eye disease, dry eye disease associated with meibomian gland dysfunction, meibomian gland dysfunction or a symptom associated therewith.

In an alternative embodiment, the composition according to the present invention, is not limited to the treatment or alleviation of dry eye or keratoconjunctivitis sicca but may also suitable for the general treatment of conditions of the eye, for example in the treatment of other inflammatory diseases, or may be used as an adjunct or additional therapy.

The inventors found that the composition according to the present invention is particularly suitable for use in a method for
i) stabilizing of the lipid layer of the tear film; and/or
ii) reducing the evaporation of underlaying water-phase of the tear film; and/or
iii) lubricating and protecting the corneal surface; and/or
iv) relief of symptoms associated with dry eye, such as burning itching, stinging, foreign body sensation.

In a further aspect, the invention relates to the composition as defined above for use in pharmaceutical methods utilizing said composition as defined above, in particular for
i) stabilizing of the lipid layer of the tear film; and/or
ii) reducing the evaporation of underlaying water-phase of the tear film; and/or
iii) lubricating and protecting the corneal surface; and/or
iv) relief of symptoms associated with dry eye, such as burning itching, stinging, foreign body sensation;
the method comprising topical administration of a composition as defined above to an eye of a subject in need thereof.

The subject may be a veterinary subject, or preferably is a human subject In the case of a human subject, in a preferred embodiment the composition is topically administered directly by the subject itself.

In a further embodiment, the invention relates to the composition as defined above for use in a method for the treatment or prevention of dry eye disease (keratoconjunctivitis sicca), evaporative dry eye disease, dry eye disease associated with meibomian gland dysfunction, Meibomian Gland Dysfunction or a symptom associated therewith comprising topically administering a composition as defined above to the eye of a subject in need thereof.

Said composition may be any composition as defined above. Preferably, the composition used in such methods is a liquid pharmaceutical composition as defined above, consisting of:
a) at least one omega-3 fatty acid ethyl ester in a total amount of up to 5 wt% based on the total weight of the composition;
b) a liquid paraffin in a total amount of up to 1 wt% based on the total weight of the composition;
c) optionally, an antioxidant in a total amount of up to 0.1 wt% or less based on the total weight of the composition,
wherein the at least one omega-3 fatty acid ethyl ester, the liquid paraffin and the optional antioxidant are dissolved in a liquid vehicle comprising a semifluorinated alkane, more preferably consisting of a semifluorinated alkane, selected from 1-perfluorohexyl-octane (F6H8) and 1-perfluorobutyl-pentane (F4H5).

Alternatively, the composition used in such methods is a liquid pharmaceutical composition as defined above, consisting of:
a) at least one omega-3 fatty acid ethyl ester in a total amount of up to 1.0 wt% based on the total weight of the composition;
b) a liquid paraffin in a total amount of up to 0.2 wt% based on the total weight of the composition;
c) optionally, an antioxidant in a total amount of up to 0.1 wt% or less based on the total weight of the composition,
wherein the at least one omega-3 fatty acid ethyl ester, the liquid paraffin and the optional antioxidant are dissolved in a liquid vehicle comprising a semifluorinated alkane, more preferably consisting of a semifluorinated alkane, selected from 1-perfluorohexyl-octane (F6H8) and 1-perfluorobutyl-pentane (F4H5).

Alternatively, the composition used in such methods is a liquid pharmaceutical composition as defined above, consisting of:
a) at least one omega-3 fatty acid ethyl ester in a total amount of up to 0.2 wt% based on the total weight of the composition;
b) a liquid paraffin in a total amount of up to 0.2 wt% based on the total weight of the composition;
c) optionally, an antioxidant in a total amount of up to 0.1 wt% or less based on the total weight of the composition,
wherein the at least one omega-3 fatty acid ethyl ester, the liquid paraffin and the optional antioxidant are dissolved in a liquid vehicle comprising a semifluorinated alkane, more preferably consisting of a semifluorinated alkane, selected from 1-perfluorohexyl-octane (F6H8) and 1-perfluorobutyl-pentane (F4H5).

The method and use require the topical application of the composition to the eye of a patient The composition is suitable for multiple applications per day. Preferably, the composition is administered to the eye of a subject in droplet form. A single dosage is preferably considered to be one single droplet per eye.

The composition is safe for ophthalmic use. In a specific embodiment of the invention, the invention relates to a pharmaceutical composition for use as defined above, wherein the composition is administered up to 4-times daily as a single droplet per eye.

Droplet size may be adjusted by use of an appropriate dispensing means and container. In general, it is preferred that the administered dose, i.e. single droplet of the composition is about, or less than 20 µl. Preferably, the droplet is about or less than 15 µL.

In a preferred embodiment, the invention relates to a composition for use as defined above, wherein the composition is administered as a single droplet of about 9 to 12 µl, preferably administering a single droplet of about 10-12 µl or 11-12 µl.

In a specific embodiment of the invention, the invention relates to a pharmaceutical composition for use as defined above, wherein the composition is administered up to 4-times daily (e.g. 1-times daily, 2-times daily, 3-times daily or 4-times daily) as a single droplet of about 10-12µl or of about 11-12 µl per eye.

The administered droplet size affects the amount of omega 3 fatty acid ethyl ester in a single dose. In one embodiment, the amount of omega-3 fatty acid ethyl ester administered in a single dose is 265 µg or less. A single droplet of defined size, preferably 20 µL or less comprises 265 µg or less of omega-3 fatty acid ethyl ester.

The administered droplet size affects the amount of omega 3 fatty acid ethyl ester in a single dose. In one embodiment, the amount of omega-3 fatty acid ethyl ester administered in a single dose is 160µg or less. A single droplet of defined size, preferably 11-12 µL or less comprises 146-160 µg or less of omega-3 fatty acid ethyl ester.

In a specific embodiment, the invention relates to a composition for use as defined above, wherein a single dose administered per eye comprises up to about 27-32 µg, preferably up to about 29-32 µg of the one or more omega-3 fatty acid ethyl esters

In a specific embodiment, the invention relates to a composition for use as defined above, wherein a daily dose administered per eye comprises up to about 106-128 µg, preferably up to about 117-128 µg, of the one or more omega-3 fatty acid ethyl esters.

In a preferred embodiment, the invention relates to a composition for use as defined above, wherein a single dose of a composition comprising 1.0 wt% docosahexaenoic acid ethyl ester administered as a single droplet of about 10-12 µl comprises about 133-160 µg docosahexaenoic acid ethyl ester

In a further preferred embodiment, the invention relates to a composition for use as defined above, wherein a single dose of a composition comprising 0.2 wt% docosahexaenoic acid ethyl ester administered as a single droplet of about 10-12 µl comprises about 27-32 µg docosahexaenoic acid ethyl ester

In a preferred embodiment, the invention relates to a composition for use as defined above, wherein a single dose of a composition comprising 1.0 wt% docosahexaenoic acid ethyl ester administered as a single droplet of about 11-12 µl comprises about 146-160 µg docosahexaenoic acid ethyl ester

In a further preferred embodiment, the invention relates to a composition for use as defined above, wherein a single dose of a composition comprising 0.2 wt% docosahexaenoic acid ethyl ester administered as a single droplet of about 11-12 µl comprises about 29-32 µg docosahexaenoic acid ethyl ester

In a preferred embodiment, the invention relates to a composition for use as defined above, wherein a daily dose of a composition comprising 1.0 wt% docosahexaenoic acid ethyl ester administered as a single droplet of about 10-12 µl comprises up to about 532-638 µg docosahexaenoic acid ethyl ester

In a further preferred embodiment, the invention relates to a composition for use as defined above, wherein a daily dose of a composition comprising 0.2 wt% docosahexaenoic acid ethyl ester administered as a single droplet of about 10-12 µl comprises up to about 106-128 µg docosahexaenoic acid ethyl ester

In a preferred embodiment, the invention relates to a composition for use as defined above, wherein a daily dose of a composition comprising 1.0 wt% docosahexaenoic acid ethyl ester administered as a single droplet of about 11-12 µl comprises up to about 585-638 µg docosahexaenoic acid ethyl ester

In a further preferred embodiment, the invention relates to a composition for use as defined above, wherein a daily dose of a composition comprising 0.2 wt% docosahexaenoic acid ethyl ester administered as a single droplet of about 11-12 µl comprises up to about 117-128µg docosahexaenoic acid ethyl ester

In a further aspect the invention relates to a method for stabilizing a composition as defined above comprising one or more omega-3 fatty acid ethyl esters comprising a step of mixing the one or more omega-3 fatty acid ethyl esters, and optionally one or more antioxidants, with a liquid paraffin and a semifluorinated alkane to form a stable clear and colorless solution; wherein the total amount of liquid paraffin based on the weight of the composition does not exceed the total amount of omega-3 fatty acid ethyl ester based on the total weight of the composition..

In a further aspect of the invention, the invention further relates to a kit comprising an ophthalmic composition as defined above. In particular the kit comprises the composition in a suitable container, preferably made from glass or plastic. The kit preferably also comprises dispensing means. The container is preferably suitable or adapted for self-administration by a subject

Accordingly, in one embodiment, the invention relates to a kit comprising a container comprising an ophthalmic composition as defined above, a dispensing means, and optionally instructions for use.

Said dispensing means are preferably adapted to allow the release of single droplets of the composition. Preferably, single droplets of 20 µL or less, preferably 15 µL or less, more preferably about 9 to 12 µl, even more preferably as a single droplet of about 10-12, more preferably as a single droplet of about 11-12 µl.

In a preferred embodiment, the invention relates to a kit as defined above, wherein the container is a glass or a plastic container. Preferably, the container is a polyethylene or polypropylene container. In specific embodiment, the container is a low-density polyethylene (LDPE) container.

In a further aspect, the invention relates to a method for stabilizing a composition as defined above comprising one or more omega-3 fatty acid ethyl esters comprising a step of mixing the one or more omega-3 fatty acid ethyl esters, and optionally one or more antioxidants, with a liquid paraffin and a semifluorinated alkane to form a stable clear and colorless solution; wherein the total amount of liquid paraffin based on the weight of the composition does not exceed the total amount of omega-3 fatty acid ethyl ester based on the total weight of the composition.

The invention further relates to a method as defined above, wherein the composition retains more than 75 wt% of omega-3 ethyl ester upon storage for 3 months at 40°c at a relative humidity of 75%.

The invention further relates to a method as defined above, wherein the composition comprises up to 5 wt% of the one or more omega-3 fatty acid ethyl esters and wherein the composition retains more than 95 wt% of omega-3 ethyl ester upon storage for 3 months at 40°C at a relative humidity of 75% in a glass container or wherein the composition retains more than 75 wt% of omega-3 ethyl ester upon storage for 3 months at 40°C at a relative humidity of 75% in a polypropylene container.

The invention further relates to a method as defined above, wherein the composition comprises up to 1 wt% of the one or more omega-3 fatty acid ethyl esters and wherein the composition retains more than 95 wt% of omega-3 ethyl ester upon storage at 40°C at a relative humidity of 75% in a glass container or wherein the composition retains more than 80 wt% of omega-3 ethyl ester upon storage at 40°C at a relative humidity of 75% in a polypropylene container.

The invention further relates to a method as defined above, wherein the composition comprises up to 0.2 wt% of the one or more omega-3 fatty acid ethyl esters and wherein the composition retains more than 95 wt% of omega-3 ethyl ester upon storage at 40°C at a relative humidity of 75% in a glass container or wherein the composition retains more than 85 wt% of omega-3 ethyl ester upon storage at 40°C at a relative humidity of 75% in a polypropylene container

The following examples serve to illustrate the invention; however, these are not to be understood as restricting the scope of the invention.

### EXAMPLES

### Example 1 - Preparation of Compositions

The following compositions were prepared by mixing the omega-3 fatty acid ethyl ester(s), antioxidant(s), with a liquid paraffin and a semifluorinated alkane as defined below to provide clear and colorless solutions.

### Composition 1

| **Component** | **Amount (mg)** | **wt %** | **10µl drop (13.3 mg)** |
|---|---|---|---|
| F6H8 | 14937.0 | 99.5800 | 13.244 mg |
| DHA-ee (docosahexaenoic acid ethyl ester) | 30.0 | 0.2 | 0.0266 mg |
| Light liquid paraffin | 30.0 | 0.2 | 0.0266 mg |
| Butylhydroxyanisole | 2.0 | 0.0133 | 0.00177 mg |
| Butylhydroxytoluene | 1.0 | 0.0067 | 0.00089 mg |

### Composition 2

| **Component** | **Amount (mg)** | **wt %** | **10µl drop (13.3 mg)** |
|---|---|---|---|
| F6H8 | 14817.0 | 98.7800 | 13.138 mg |
| DHA-ee (docosahexaenoic acid ethyl ester) | 30.0 | 0.2 | 0.0266 mg |
| Light liquid paraffin | 150.0 | 1.0 | 0.133 mg |
| Butylhydroxyanisole | 2.0 | 0.0133 | 0.00177 mg |
| Butylhydroxytoluene | 1.0 | 0.0067 | 0.00089 mg |

### Composition 3

| **Component** | **Amount (mg)** | **wt %** | **10µl drop (13.3 mg)** |
|---|---|---|---|
| F6H8 | 14817.0 | 99.5800 | 13.244 mg |
| DHA-ee, EPA-ee Mixture (43:57) | 30.0 (31.3) | 0.2 | 0.0266 mg |
| Light liquid paraffin | 30.0 | 0.2 | 0.0266 mg |
| Butylhydroxyanisole | 2.0 | 0.0133 | 0.00177 mg |
| Butylhydroxytoluene | 1.0 | 0.0067 | 0.00089 mg |

### Composition 4

| **Component** | **Amount (mg)** | **wt %** | **Ca. 11.6µl drop (ca. 15.5 mg)** |
|---|---|---|---|
| F6H8 | 14937 | 99.5800 | 15.4349 |
| DHA-ee (docosahexaenoic acid ethyl ester) | 30 | 0.2 | 0.031 |
| Light Liquid paraffin | 30 | 0.2 | 0.031 |
| Butylhydroxyanisole | 2 | 0.0133 | 0.002015 |
| Butylhydroxytoluene | 1 | 0.0067 | 0.001085 |
| Total | 15000.0 | 100 | 15.5 |

### Example 2 - Formulation Stability

The following formulations were tested for stability for 6 weeks at 40°C/75%RH in glass vials:
- 0.5 wt% EPA-ee, 0.001 wt% alpha-tocopherol in F6H8 (not according to the invention)
- 0.5 wt% EPA-ee, 0.001 wt% alpha-tocopherol in F6H8, 0.5 wt% light liquid paraffin
- 0.5 wt% EPA-ee, 0.001 wt% alpha-tocopherol in F6H8, 10 wt% light liquid paraffin (not according to the invention)

Upon preparation in glass vials, all formulations presented as single-phase clear colorless solutions. Testing after 6 weeks at 50°C revealed that no solid deposition, such as tidemark formation was observed for EPA-ee formulations in F6H8 comprising light liquid paraffin, whereas a yellow tidemark on the glass wall at the interphase between the liquid surface and the headspace was clearly observed for the formulation without light liquid paraffin. This demonstrated that the addition of light liquid paraffin prevented the formation of a solid deposition, such as a tidemark, when compared to formulations prepared without addition of light liquid paraffin.

The following formulation was tested for solid deposition formation, such as tidemark formation in a 5-mL, white polypropylene (PP) eye dropper bottles fitted with a low-density polyethylene (LDPE) dropper and a high-density polyethylene (HDPE) screw-on cap:
- 0.5 wt% DHA-ee, 0.001 wt% alpha-tocopherol in F4H5 (not according to the invention)

The samples were prepared as clear colorless solutions at a volume of 3 ml and stored for 6 months at controlled conditions of 40°C/75% RH. After 6 months, the bottles were emptied and carefully inspected. Visual inspection revealed an intense yellow solid deposition, namely a tidemark on the inner walls of the PP eye dropper bottle.

The yellow solid deposition, in form of a tidemark, was then washed by shaking the bottle with 3 ml of ethanol absolute. The ethanolic wash solution was then analyzed by GC-FID on identity and content of DHA-ee. It was found that the ethanolic wash solution contained 5% of the nominal DHA-ee content. This result confirmed that omega-3 ethyl ester component of ophthalmic solution is being adsorbed over time to the material of the primary packaging (i.e. the inner container wall of an eyedropper bottle).

### Example 3 - Absorption to polypropylene

This experiment was performed to investigate the absorption phenomena that occurred for solutions of Omega-3 ethyl esters in a semifluorinated alkane (SFA)stored in polypropylene bottles.

The compositions consist of 3 mL 5 wt% solution of docosahexaenoic acid ethyl ester (DHA-ee) in F4H5 (1-perfluorobutyl-pentane) and F6H8 (1-perfluorohexyl-octane) filled in 5 mL polypropylene (PP) bottles fitted with an LDPE dropper and an HDPE screw on cap. After preparation the compositions were stored at controlled conditions 40°C and 75% humidity for 6 months.

| | |
|---|---|
| Composition A: | 5.0 wt% DHA-ee |
| | 0.20 wt% light liquid paraffin |
| | 0.02 wt% BHA/BHT (0.0133 wt% butylhydroxyanisol, and 0.0067 wt% butyl hydroxy toluene) |
| | dissolved in F6H8 |
| Composition B (not according to the invention): | 5.0 wt% DHA-ee |
| | dissolved in F4H5 |

After 6 months the samples were taken from the 40°C/75% RH chamber, the bottles were emptied of their content and the solutions were transferred to a 5 mL clear glass vial and stored at RT. Visual inspection of the recovered solutions revealed that the composition comprising light liquid paraffin (A) had a light yellowish color, while the one without light liquid paraffin (B) did show an intense yellow color. Determination of the concentration of both solutions confirmed that the content of DHA-ee was significantly reduced by ∼58% in the composition comprising no light liquid paraffin (B), while the content of DHA-ee in composition (A) did still meet the stability acceptance criteria (minimum: 75 wt%; maximum : 120 wt%).

Afterwards, the empty polypropylene bottles were then filled with 3 ml of ethanol absolute and placed on a lab shaker set at 280 rpm over the weekend at room temperature. Then, the samples were withdrawn from the shaker and the wash solutions were transferred into 5 mL glass vials. A visual inspection of the wash solution was conducted. The wash solution originating from the bottle previously containing composition (B), which did not contain light liquid paraffin was brown in color, whereas the wash solution derived from the bottle previously containing composition (A) including the light liquid paraffin was yellow in color. Determination of the DHA-ee concentration in both ethanolic wash solutions resulted in concentrations of about 0.24 wt% of DHA-ee, translating to 5 % of the initial nominal DHA-ee concentration (baseline). The results are summarized in the following table.

| **Composition** | **Storage condition** | **DHA-ee, baseline (bottle, solution)** | **DHA-ee, after 6 months (bottle, solution)** | **DHA-ee, after 6 months (bottle, wall - after EtOH-wash)** |
|---|---|---|---|---|
| 5.0 wt% DHA-ee | 6 months at 40°C/75% RH | 5.0 (100%) | 4.376 (88%) | 0.244 (5%) |
| 0.02 wt% BHA/BHT | | | | |
| 0.20 wt% light liquid paraffin dissolved in F6H8 **(A)** | | | | |
| 5.0 wt% DHA-ee dissolved in F4H5 **(B)** | 6 months at 40°C/75% RH | 5.0 (100%) | 2.091 (42%) | 0.242 (5%) |

These results clearly confirmed that a portion of the omega-3 ethyl ester component is being adsorbed over time to the plastic material (polypropylene) of the container and that this absorption can be effectively suppressed by addition of light liquid paraffin. Without being bound by theory, it is believed that the light liquid paraffin passivates the polypropylene by adhering to the polypropylene wall of the container itself, thereby effectively blocking the omega-3 ethyl ester from adsorbing to the plastic material.

### Example 4 - Stability Study

The study was performed to investigate the stability of a compositions comprising omega-3 fatty acids ethyl esters, light liquid paraffin, dissolved in semifluorinated alkanes (F4H5, F6H8). Omega-3 fatty acids as their ethyl esters were obtained from plant oil origin (algae) or of fish oil origin from qualified suppliers. For stabilization of the omega-3 fatty acids ethyl esters light liquid paraffin was added. The product is intended for the use as eye drops. The study is executed with 5 mL containers (glass vials, polypropylene vials) containing 1 mL of the respective compositions. No antioxidant is added to the compositions **1-10.**

The study samples included:

| **No.** | **Omega-3** | **Omega-3, source** | **Omega-3, conc.** | **light liquid paraffin** | **SFA** | **container** |
|---|---|---|---|---|---|---|
| **1** | DHA-ee | algae | 5wt% | 1 wt% | F6H8 | glass |
| **2** | DHA-ee | algae | 1 wt% | 1 wt% | F6H8 | glass |
| **3** | DHA-ee | algae | 0.2 wt% | 0.2 wt% | F6H8 | glass |
| **4** | DHA-ee | algae | 1 wt% | 1 wt% | F4H5 | polypropylene |
| **5** | DHA-ee | algae | 0.2 wt% | 0.2 wt% | F4H5 | polypropylene |
| **6** | DHA-ee/EPA-ee | fish oil | 5 wt% | 1 wt% | F4H5 | polypropylene |
| **7** | DHA-ee/EPA-ee | fish oil | 1 wt% | 1 wt% | F4H5 | polypropylene |
| **8** | DHA-ee/EPA-ee | fish oil | 0.2 wt% | 0.2 wt% | F4H5 | polypropylene |
| **9** | DHA-ee | algae | 1 wt% | 1 wt% | F6H8 | polypropylene |
| **10** | DHA-ee | algae | 0.2 wt% | 0.2 wt% | F6H8 | polypropylene |

with: DHA-ee = docosahexaenoic acid ethyl ester; EPA-ee = eicosapentaenoic acid ethyl ester; DHA-ee/EPA-ee = mixture of DHA-ee & EPA-ee (43:57).

The samples were stored under ICH accelerated condition (40°C/75% RH). For omega-3-ethyl ester assay and identity determination, 250 µL of the respective solution was transferred into an amber glass auto sampler vial for GC-FID analysis. The combined omega-3 ethyl esters assay and identification test was based on a GC-FID procedure with a validated method with quantitation based on an external standard calibration. In this study stability was assessed in terms of the observed decreases of omega-3 fatty acids ethyl ester wt% over time. Results of the stability study are shown in Figures 1 (A)-1(D) and the following tables.

### A) Omega-3 source: algae; container: glass vials

The table below and Figure 1A demonstrate the stability of compositions comprising docosahexaenoic acid ethyl ester (DHA-ee), 0.2 wt% light liquid paraffin, dissolved in F6H8 **(1, 2, 3).** The stability of the compositions stored in glass vials over a period of 3 months at 40°C at 75% humidity (ICH)was assessed at monthly intervals. The stability acceptance criteria (minimum: 75 wt%; maximum: 120 wt%) were met.

| **ICH 40°C/75% RH** | **T0** | **M1** | **M2** | **M3** |
|---|---|---|---|---|
| 5 wt% DHA-ee/F6H8 **(1)** | 100.0% | 96.6% | 96.6% | 100.8% |
| 1 wt% DHA-ee/F6H8 **(2)** | 100.0% | 92.8% | 92.8% | 96.7% |
| 0.2 wt% DHA-ee/F6H8 **(3)** | 100.0% | 95.5% | 95.5% | 98.5% |

### B) Omega-3 source: algae; container: polypropylene vials

The table below and Figure 1B demonstrate the stability of compositions comprising docosahexaenoic acid ethyl ester, 0.2 wt% light liquid paraffin, dissolved in F4H5 **(4, 5).** The stability of the compositions stored in polypropylene vials a period of 3 months at 40°C at 75% humidity (ICH)was assessed at monthly intervals. The stability acceptance criteria (minimum: 75 wt%; maximum: 120 wt%) were met.

| **ICH 40°C/75% RH** | **T0** | **M1** | **M2** | **M3** |
|---|---|---|---|---|
| 1 wt% DHA-ee/F4H5 **(4)** | 100.0% | 91.7% | 90.6% | 92.2% |
| 0.2 wt% DHA-ee/F4H5 **(5)** | 100.0% | 89.5% | 94.7% | 95.3% |

### C) Omega-3 source: fish oil; container: polypropylene vials

The table below and Figure 1C demonstrate the stability of compositions comprising a mixture (43:57) of docosahexaenoic acid ethyl ester (DHA-ee) and eicosapentaenoic acid ethyl ester (EPA-ee), 0.2 wt% light liquid paraffin, dissolved in F4H5 **(6, 7, 8).** The stability of the compositions stored in polypropylene vials over a period of 3 months at 40°C at 75% humidity (ICH) was assessed at monthly intervals. The stability acceptance criteria (minimum: 75 wt%; maximum: 120 wt%) were met.

| **ICH 40°C/75% RH** | **T0** | **M1** | **M2** | **M3** |
|---|---|---|---|---|
| 5 wt% DHA-ee, EPA-ee (43:57) /F4H5 **(6)** | 100.0% | 86.1% | 80.0% | 77.3% |
| 1 wt% DHA-ee, EPA-ee (43:57) /F4H5 **(7)** | 100.0% | 92.9% | 85.9% | 84.8% |
| 0.2 wt% DHA-ee, EPA-ee (43:57) /F4H5 **(8)** | 100.0% | 94.7% | 89.5% | 89.5% |

### (D) Omega-3 source: algae; polypropylene vials

The table below and Figure 1D demonstrates the stability of compositions comprising docosahexaenoic acid ethyl ester and, 0.2 wt% light liquid paraffin, dissolved in F6H8 **(9, 10**). The stability of the compositions stored in in polypropylene vials over a period of 3 months at 40°C at 75% humidity (ICH) was assessed at monthly intervals. The stability acceptance criteria (minimum: 75 wt%; maximum: 120 wt%) were met

| **ICH 40°C/75% RH** | **T0** | **M1** | **M2** | **M3** |
|---|---|---|---|---|
| 1 wt% DHA-ee/F6H8 **(9)** | 100.0% | 88.9% | 85.9% | 89.2% |
| 0.2 wt% DHA-ee/F6H8 **(10)** | 100.0% | 90.0% | 90.0% | 94.5% |

The stability study shows that the stability of compositions containing omega-3 fatty acid ethyl ester and semifluorinated alkanes can be significantly improved and the adsorption of the omega-3 fatty acid ethyl esters to the vial can be reduced by the addition of light liquid paraffin to the compositions independently of the addition of antioxidants.

### Example 5 - Oxygen content measurement

The oxygen content of the composition was determined using a chemical optical oxygen microsensor (PreSens - Precision Sensing GmbH; Regensburg, Germany). The measuring range for the microsensor is 0 - 250 % air saturation, which translates to 0 - 22.5 mg/L dissolved oxygen.

| **Component** | **wt %** | **Component** | **wt %** |
|---|---|---|---|
| F6H8 | 99.5800 | F6H8 | 99.5800 |
| DHA-ee (docosahexaenoic acid ethyl ester) | 0.2 | DHA-ee, EPA-ee Mixture (43:57) | 0.2 |
| Light liquid paraffin | 0.2 | Light liquid paraffin | 0.2 |
| Butylhydroxyanisole | 0.0133 | Butylhydroxyanisole | 0.0133 |
| Butylhydroxytoluene | 0.0067 | Butylhydroxytoluene | 0.0067 |
| Oxygen saturation | < 5% | Oxygen saturation | <5% |
| Oxygen content | < 0.45 µg/ml | Oxygen content | < 0.45 µg/ml |

## Claims

1. A stable liquid pharmaceutical composition comprising:
a) at least one omega-3 fatty acid ethyl ester in a total amount of up to 5 wt% based on the total weight of the composition;
b) a liquid paraffin in a total amount of up to 1 wt% based on the total weight of the composition;
wherein the at least one omega-3 fatty acid ethyl ester and the liquid paraffin are dissolved in a liquid vehicle comprising a semifluorinated alkane selected from 1-perfluorohexyl-octane (F6H8) or 1-perfluorobutyl-pentane (F4H5);
wherein the liquid paraffin is light liquid paraffin.

2. The composition according to claim 1, wherein the total amount of liquid paraffin in wt% does not exceed the total amount of omega-3 fatty acid ethyl ester in wt%.

3. The composition according to claim 1 or 2, wherein the at least one omega 3 fatty acid ethyl ester is eicosapentaenoic acid ethyl ester, docosahexaenoic acid ethyl ester or a mixture thereof.

4. The composition according to any one of the preceding claims, wherein the composition substantially consists of at least one omega-3 fatty acid ethyl ester and a liquid paraffin dissolved in a semifluorinated alkane and optionally an antioxidant.

5. The composition according to any one of the claims 1 to 4, comprising or consisting of up to 0.2 wt% of an omega-3 fatty acid ethyl ester selected from docosahexaenoic acid ethyl ester, eicosapentaenoic acid ethyl ester or mixtures thereof, 0.2 wt% light liquid paraffin, dissolved in a liquid vehicle comprising a semifluorinated alkane selected from F4H5, F6H8 and mixtures thereof and optionally up to 0.1 wt% of antioxidant.

6. The composition according to any of the claims 1 to 4, comprising up to 0.2 wt% docosahexaenoic acid ethyl ester, 0.2 wt% light liquid paraffin, at least 99 wt% of a semifluorinated alkane selected from F4H5, F6H8 and mixtures thereof, and optionally up to 0.1 wt% of antioxidant.

7. The composition according to any of the claims 1 to 4, essentially consisting of 0.2 wt% docosahexaenoic acid ethyl ester, 0.2 wt% light liquid paraffin, dissolved in a semifluorinated alkane selected from F4H5, F6H8 and mixtures thereof and optionally up to 0.1 wt% of antioxidant.

8. The composition according to any of the claims 1 to 4, essentially consisting of 0.2 wt% docosahexaenoic acid ethyl ester, 0.2 wt% light liquid paraffin, 0.0133 wt% butylhydroxyanisol, and 0.0067 wt% butylhydroxytoluene dissolved in F6H8.

9. The composition according to any one of the preceding claims, wherein the composition retains more than 75 wt% for 3 months of omega-3 ethyl ester upon storage at 40°c at a relative humidity of 75%.

10. The composition according to any preceding claim, wherein the oxygen content of the composition is below 0.90 µg/ml.

11. The composition according to any one of the preceding claims, wherein the composition is an ophthalmic composition.

12. The composition according to any one of the preceding claims for use as a medicament.

13. The composition for use according to claim 12, wherein the composition is topically administered, preferably the composition is topically administered to the eye, to an eye surface, to the lacrimal sac, to the upper or lower eyelid or to an ophthalmic tissue.

14. The composition for use according to claim 12 or 13, for use in the prevention or treatment of dry eye disease (keratoconjunctivitis sicca), evaporative dry eye disease, dry eye disease associated with Meibomian Gland Dysfunction, Meibomian Gland Dysfunction or a symptom or condition associated therewith.

15. A kit comprising a container comprising an ophthalmic composition according to claim 11, a dispensing means, and optionally instructions for use.

16. A method for stabilizing a composition comprising one or more omega-3 fatty acid ethyl esters as defined in any of the claims 1 to 11, comprising a step of mixing the one or more omega-3 fatty acid ethyl esters, a liquid paraffin and optionally one or more antioxidants, with a semifluorinated alkane to form a stable clear and colorless solution.

## Patentansprüche

1. Stabile flüssige pharmazeutische Zusammensetzung, umfassend:
a) mindestens einen Omega-3-fettsäureethylester in einer Gesamtmenge von bis zu 5 Gew.-%, bezogen auf das Gesamtgewicht der Zusammensetzung,
b) ein flüssiges Paraffin in einer Gesamtmenge von bis zu 1 Gew.-%, bezogen auf das Gesamtgewicht der Zusammensetzung,
wobei der mindestens eine Omega-3-fettsäureethylester und das flüssige Paraffin in einem flüssigen Vehikel gelöst sind, das ein semifluoriertes Alkan ausgewählt aus 1-Perfluorhexyloctan (F6H8) und 1-Perfluorbutylpentan (F4H5) umfasst,
wobei es sich bei dem flüssigen Paraffin um Weißöl handelt.

2. Zusammensetzung nach Anspruch 1, wobei die Gesamtmenge an flüssigem Paraffin in Gew.-% nicht über der Gesamtmenge an Omega-3-fettsäureethylester in Gew.-% liegt.

3. Zusammensetzung nach Anspruch 1 oder 2, wobei es sich bei dem mindestens einen Omega-3-fettsäureethylester um Eicosapentaensäureethylester, Docosahexaensäureethylester oder eine Mischung davon handelt.

4. Zusammensetzung nach einem der vorhergehenden Ansprüche, wobei die Zusammensetzung im Wesentlichen aus mindestens einem Omega-3-fettsäureethylester und einem flüssigen Paraffin gelöst in einem semifluorierten Alkan und gegebenenfalls einem Antioxidationsmittel besteht.

5. Zusammensetzung nach einem der Ansprüche 1 bis 4, umfassend oder bestehend aus bis zu 0,2 Gew.-% eines Omega-3-fettsäureethylesters ausgewählt aus Docosahexaensäureethylester, Eicosapentaensäureethylester und Mischungen davon, 0,2 Gew.-% Weißöl, gelöst in einem flüssigen Vehikel, das ein semifluoriertes Alkan ausgewählt aus F4H5, F6H8 und Mischungen davon umfasst, und gegebenenfalls bis zu 0,1 Gew.-% Antioxidationsmittel.

6. Zusammensetzung nach einem der Ansprüche 1 bis 4, umfassend bis zu 0,2 Gew.-% Docosahexaensäureethylester, 0,2 Gew.-% Weißöl, mindestens 99 Gew.-% eines semifluorierten Alkans ausgewählt aus F4H5, F6H8 und Mischungen davon und gegebenenfalls bis zu 0,1 Gew.-% Antioxidationsmittel.

7. Zusammensetzung nach einem der Ansprüche 1 bis 4, im Wesentlichen bestehend aus 0,2 Gew.-% Docosahexaensäureethylester, 0,2 Gew.-% Weißöl, gelöst in einem semifluorierten Alkan ausgewählt aus F4H5, F6H8 und Mischungen davon, und gegebenenfalls bis zu 0,1 Gew.-% Antioxidationsmittel.

8. Zusammensetzung nach einem der Ansprüche 1 bis 4, im Wesentlichen bestehend aus 0,2 Gew.-% Docosahexaensäureethylester, 0,2 Gew.-% Weißöl, 0,0133 Gew.-% Butylhydroxyanisol und 0,0067 Gew.-% Butylhydroxytoluol gelöst in F6H8.

9. Zusammensetzung nach einem der vorhergehenden Ansprüche, wobei die Zusammensetzung bei Lagerung bei 40 °C bei einer relativen Luftfeuchtigkeit von 75 % über 3 Monate mehr als 75 Gew.-% Omega-3-ethylester beibehält.

10. Zusammensetzung nach einem der vorhergehenden Ansprüche, wobei der Sauerstoffgehalt der Zusammensetzung unter 0,90 µg/ml liegt.

11. Zusammensetzung nach einem der vorhergehenden Ansprüche, wobei die Zusammensetzung eine ophthalmische Zusammensetzung ist.

12. Zusammensetzung nach einem der vorhergehenden Ansprüche zur Verwendung als Medikament.

13. Zusammensetzung zur Verwendung nach Anspruch 12, wobei die Zusammensetzung topisch verabreicht wird, wobei die Zusammensetzung vorzugsweise topisch an das Auge, an eine Augenoberfläche, an den Tränensack, an das obere oder untere Augenlid oder an ein Augengewebe verabreicht wird.

14. Zusammensetzung zur Verwendung nach Anspruch 12 oder 13 zur Verwendung bei der Prävention oder Behandlung des Syndroms des trockenen Auges (Keratokonjunktivitis sicca), Syndroms des trockenen Auges durch Verdunstung, Syndroms des trockenen Auges in Verbindung mit Meibom-Drüsen-Dysfunktion, Meibom-Drüsen-Dysfunktion oder einem damit assoziierten Symptom oder Leiden.

15. Kit, umfassend einen Behälter mit einer ophthalmischen Zusammensetzung nach Ansprüche 11, eine Abgabevorrichtung und gegebenenfalls eine Gebrauchsanweisung.

16. Verfahren zum Stabilisieren einer oder mehrerer wie in einem der Ansprüche 1 bis 11 definierten Omega-3-fettsäureethylester, umfassend den Schritt des Mischens des einen oder der mehreren Omega-3-fettsäureethylester, eines flüssigen Paraffins und gegebenenfalls eines oder mehrerer Antioxidationsmittel mit einem semifluorierten Alkan unter Bildung einer stabilen klaren und farblosen Lösung.

## Revendications

1. Composition pharmaceutique liquide stable comprenant :
a) au moins un ester éthylique d'acide gras oméga-3 dans une quantité totale allant jusqu'à 5 % en poids sur la base du poids total de la composition ;
b) une paraffine liquide dans une quantité totale allant jusqu'à 1 % en poids sur la base du poids total de la composition ;
l'au moins un ester éthylique d'acide gras oméga-3 et la paraffine liquide étant dissous dans un véhicule liquide comprenant un alcane semi-fluoré choisi parmi le 1-perfluorohexyl-octane (F6H8) ou le 1-perfluorobutyl-pentane (F4H5) ;
la paraffine liquide étant de la paraffine liquide légère.

2. Composition selon la revendication 1, la quantité totale de paraffine liquide en % en poids ne dépassant pas la quantité totale d'ester éthylique d'acide gras oméga-3 en % en poids.

3. Composition selon la revendication 1 ou 2, l'ester éthylique d'acide gras oméga 3 étant l'ester éthylique d'acide eicosapentaénoïque, l'ester éthylique d'acide docosahexaénoïque ou un mélange de ceux-ci.

4. Composition selon l'une quelconque des revendications précédentes, la composition étant constituée dans l'ensemble d'au moins un ester éthylique d'acide gras oméga-3 et d'une paraffine liquide dissoute dans un alcane semi-fluoré et éventuellement d'un antioxydant.

5. Composition selon l'une quelconque des revendications 1 à 4, comprenant ou étant constitué jusqu'à 0,2 % en poids d'un ester éthylique d'acide gras oméga-3 choisi parmi l'ester éthylique d'acide docosahexaénoïque, l'ester éthylique d'acide eicosapentaénoïque ou des mélanges de ceux-ci, 0,2 % en poids de paraffine liquide légère, dissoute dans un véhicule liquide comprenant un alcane semi-fluoré choisi parmi F4H5, F6H8 et leurs mélanges et éventuellement jusqu'à 0,1 % en poids d'antioxydant.

6. Composition selon l'une quelconque des revendications 1 à 4, comprenant jusqu'à 0,2 % en poids d'ester éthylique d'acide docosahexaénoïque, 0,2 % en poids de paraffine liquide légère, au moins 99 % en poids d'un alcane semi-fluoré choisi parmi F4H5, F6H8 et leurs mélanges, et éventuellement jusqu'à 0,1 % en poids d'antioxydant.

7. Composition selon l'une quelconque des revendications 1 à 4, constituée essentiellement de 0,2 % en poids d'ester éthylique d'acide docosahexaénoïque, 0,2 % en poids de paraffine liquide légère, dissoute dans un alcane semi-fluoré choisi parmi F4H5, F6H8 et leurs mélanges et éventuellement jusqu'à 0,1 % en poids d'antioxydant.

8. Composition selon l'une quelconque des revendications 1 à 4, constituée essentiellement de 0,2 % en poids d'ester éthylique d'acide docosahexaénoïque, 0,2 % en poids de paraffine liquide légère, 0,0133 % en poids de butylhydroxyanisol et 0,0067 % en poids de butylhydroxytoluène dissous dans F6H8.

9. Composition selon l'une quelconque des revendications précédentes, la composition retenant plus de 75 % en poids pendant 3 mois d'ester éthylique d'oméga-3 lors d'un stockage à 40 °C à une humidité relative de 75 %.

10. Composition selon l'une quelconque des revendications précédentes, la teneur en oxygène de la composition étant inférieure à 0,90 µg/ml.

11. Composition selon l'une des revendications précédentes, dans laquelle la composition est une composition ophtalmique.

12. Composition selon l'une quelconque des revendications précédentes destinée à être utilisée comme médicament.

13. Composition destinée à être utilisée selon la revendication 12, la composition étant administrée par voie topique, de préférence la composition étant administrée par voie topique à l'oeil, à une surface de l'oeil, au sac lacrymal, à la paupière supérieure ou inférieure ou à un tissu ophtalmique.

14. Composition destinée à être utilisée selon la revendication 12 ou 13, à être utilisée dans la prévention ou le traitement de la sécheresse oculaire (kératoconjonctivite sicca), la sécheresse oculaire par évaporation, la sécheresse oculaire associée à un dysfonctionnement de la glande de Meibomius, un dysfonctionnement de la glande de Meibomius ou un symptôme ou une affection associée.

15. Kit comprenant un récipient contenant une composition ophtalmique selon la revendication 11, un moyen de distribution, et éventuellement un mode d'emploi.

16. Procédé de stabilisation d'une composition comprenant un ou plusieurs esters éthyliques d'acide gras oméga-3 tels que définis dans l'une quelconque des revendications 1 à 11, ledit procédé comprenant une étape de mélange des un ou plusieurs esters éthyliques d'acide gras oméga-3, d'une paraffine liquide et éventuellement des un ou plusieurs antioxydants, à un alcane semi-fluoré pour former une solution stable, claire et incolore.
